Europäisches Patentamt

European Patent Office

Office européen des brevets

(11) Publication number: **0 144 316**
**B1**

# (12) EUROPEAN PATENT SPECIFICATION

(45) Date of publication of patent specification: **22.02.89**

(51) Int. Cl.⁴: **A 61 B 6/00**

(21) Application number: **83902200.1**

(22) Date of filing: **31.05.83**

(86) International application number:
**PCT/US83/00864**

(87) International publication number:
**WO 84/04664 06.12.84 Gazette 84/28**

(54) ANGIOGRAPHIC CATHETER WITH SOFT TIP END.

| | |
|---|---|
| (43) Date of publication of application:<br>**19.06.85 Bulletin 85/25** | (73) Proprietor: **SHERWOOD MEDICAL COMPANY**<br>**1831 Olive Street**<br>**St. Louis, MO 63103 (US)** |
| (45) Publication of the grant of the patent:<br>**22.02.89 Bulletin 89/08** | (72) Inventor: **Ruiz, Oscar Francisco**<br>**3655 Bay Homes Drive**<br>**Coconut Grove Florida 33133 (US)** |
| (84) Designated Contracting States:<br>**AT BE CH DE FR GB LI LU NL SE** | (74) Representative: **Miller, Joseph et al**<br>**J. MILLER & CO. Lincoln House 296-302 High**<br>**Holborn**<br>**London WC1V 7JH (GB)** |
| (56) References cited:<br>**DE-A-2 140 755**<br>**FR-A-2 188 448**<br>**US-A-2 857 915**<br>**US-A-3 612 038**<br>**US-A-3 618 614**<br>**US-A-3 935 857**<br>**US-A-4 015 601**<br>**US-A-4 117 836**<br>**US-A-4 184 497**<br>**US-A-4 279 252**<br>**US-A-4 282 876** | |

Courier Press, Leamington Spa, England.

## Description

This invention relates to an angiographic catheter.

In the past there have been various types of angiographic catheters. Generally speaking, such cathethers are utilized for diagnostic purposes. More particularly, such a catheter is inserted into an artery or vein and advanced in a generally axial direction through the vessel to a predetermined site as determined by an attending physician for the purpose of injecting radio opaque material. The radio opaque material has to be injected under a relatively high pressure in order to achieve a relatively high concentration in a short period of time so that the images which are achieved in the conventional manner are as sharp as possible.

It will be appreciated by those in the art that such catheters must be relatively rigid so as to be capable of advancement by pushing, to be susceptible to axial movements of adjustment and to resist twisting or torque forces. Such catheters must also be able to withstand a high bursting pressure while, at the same time, the tip should be so formed as not to cause injury to the interior wall of the vessel as it is advanced.

The prior art includes braided angiographic catheters, that is catheters which are reinforced with a steel wire mesh or Dacron reinforcing braid in order to accommodate high compressive forces and resistance to bursting, as required to move it through a vein to a desired site and deliver radio opaque dye under relatively high pressures. There have also been other types of angiographic catheters, for example, two tubes, each of a different type of polyethylene and each of a different rigidity have been adhered together in coaxial relation.

US Patent 4,282,876 discloses such an angiographic catheter comprising an inner tube jacketed by an outer tube and having an overall wall thickness substantially common throughout the axial length of the catheter. The catheter described in this earlier US Patent has a multiwall tube of co-taper construction with the inner tube being softer than the outer jacket and has a degree of flexibility which progressively changes along the length of the catheter.

The technical problem on which the present invention is based is that of providing an angiographic catheter having a terminal end or leading end, which is soft, so that it will not penetrate a vessel wall, or traumatize the vessel, and which will be less likely than known catheters to dislodge plaque from the interior wall of a vessel through which it is being advanced, yet which is nevertheless sufficiently reinforced as to have a high bursting strength and be capable of resisting compressive forces so that it can be axially advanced and rotated within a vein as described above.

According to the present invention, therefore, there is provided an angiographic catheter comprising a composite tube formed by an inner sleeve surrounded by an outer sleeve co-tapered therewith over a part of the length of the catheter such that the overall wall thickness of the composite tube is substantially constant throughout its axial length, characterised in that the inner sleeve is composed of a reinforcing polyamide material jacketed by the outer sleeve of soft elastomeric material, in that the outer sleeve extends beyond a tapered end of the reinforcing polyamide inner sleeve to form an unreinforced distal end portion of soft elastomeric urethane constituting a soft tip, and in that the tapered end of the reinforcing polyamide inner sleeve defines an intermediate zone of decreasing stiffness from a main reinforced length of the catheter to the unreinforced distal end portion.

This invention offers the advantage of providing an improved reinforced angiographic catheter having a relatively soft tip so as to be, generally speaking, non-traumatic. This invention also has the advantage of providing an improved reinforced angiographic catheter having an exterior sleeve or surface of a soft elastomeric material and a relatively soft non-traumatic tip which is not reinforced.

An angiographic catheter formed according to the invention may have a soft tip of a length between 3 and 7 millimeters and preferably about 5 millimeters which is non-reinforced and is of flexible, pliable, yieldable, bendable material.

The present invention thus provides an improved soft non-traumatic tipped angiographic catheter which is generally inexpensive to manufacture, which is capable of injecting radio opaque material under high pressure and which, whilst having relatively thin walls will nevertheless not burst at pressures of up to about $8.2737 \times 10^7$ Pa (1,200 psi) or higher for the purposes which are set forth more fully hereinafter.

One embodiment of the present invention will now be more particularly described by way of example, with reference to the accompanying drawings, in which:

Figure 1 is a side view of the terminal end zone of an angiographic catheter formed in accordance with this invention; and

Figure 2 is an enlarged view of a part of the catheter shown in Figure 1 taken on the plane indicated by the arrowed lines designated by the numerals 2—2.

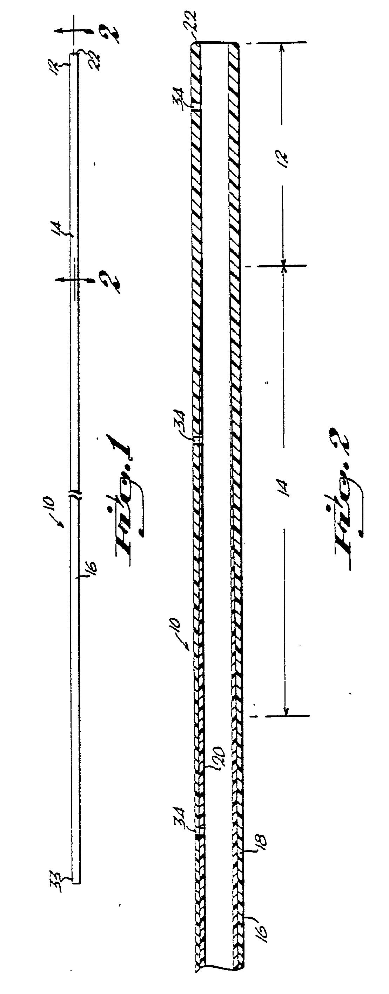

Referring to the drawings the improved catheter is generally designated by the numeral 10. As seen in Figure 2, it includes a soft tip or a terminal zone designated by the numeral 12, an intermediate zone 14, and a main fully reinforced length 16. Along the entire length there is an exterior jacket or sleeve designated by the numeral 18 of elastomeric material which is relatively soft, bendable, flexible and pliable. Within the main length of this jacket, as designated by the numeral 20, there is a reinforcing tube adhered to the inside diameter of the elastomeric jacket or sleeve. This inner tube is of a polyamide, for example nylon, which provides rigidity, torque control, and may generally be described as being of high strength, so that, when the catheter is pushed axially, it will advance in response to the applied forces without collapsing and offers sufficient strength that dye can be forced through it under high pressure without bursting the catheter.

Between the terminal end zone 12 and the main length 16, there is an intermediate zone 14, see Figure 2. In this intermediate zone, the inner and outer tubes are co-tapered, so that the outer sleeve of elastomeric material is enlarged in cross-section at the distal portion of this zone, as compared to the proximal portion, while, correspondingly, the relatively rigid interior tube of reinforcing polyamide is tapered to provide an easy transition between the main fully reinforced length and the soft tip.

As is perhaps well known, the inside diameter of an angiographic catheter should be in the range of 0.5 millimetres (0.020 inches) to about 1.77 millimetres (0.070 inches). This structure as described above accommodates such interior diameter while the outer circumference may be held to between 2 millimeters and 8 millimeters. It will be appreciated that this construction provides a relatively thin wall while maintaining a large lumen with high pressure bearing capability.

In the preferred embodiment shown, the intermediate zone designated by the numeral 14 is between 1 and 3 centimeters in length. In the preferred embodiment, throughout the intermediate zone, the taper of the reinforcing polyamide is uniform and goes to zero at the beginning of the tip zone 12, that is, it reaches zero thickness. In the intermediate zone, the sum of the wall thickness of the soft outer elastomeric material plus the wall thickness of the reinforcing material is about the same as that of the main length 16 and of the elastomeric tip 12, which is not reinforced; however, the latter may be somewhat thinner or thicker. The length of the tip zone 12 is preferably in the range of between 1 millimeter and 7 millimeters.

It will be appreciated that, if the end 22 of the catheter were of generally as high strength as the main length 16, then as the leading end or tip is advanced through a vessel, it might pierce or cause trauma to the interior wall of the vessel at a bend or curve. The construction of the present invention thus provides a terminal end zone which is soft, bendable, flexible and pliable and which can be advanced readily and utilized in the manner described.

It is thus seen that there is provided a soft tip angiographic catheter which is able to withstand an interior bursting pressure of up to $8.2737 \times 10^7$ Pa (1,200 psi) so that a high concentration of radio opaque material may be injected in a short time period and a clear picture taken for a diagnostic purpose by an attending physician. This is accomplished by the structure disclosed without the necessity of braiding or steel reinforcing wire mesh which have been utilized in the past. When such reinforcing braiding has been used in the past it has sometimes become broken, dislodged or separated causing internal damage and had to be retrieved by a snare or surgically.

It will be understood that the proximal end 33 of the catheter is adapted in a conventional manner for connection to a standard Luer Lock or hub for introducing the dye from a source; and that a pattern of holes such as 34 may be provided as desired along the length or just at the tip or tip and intermediate zone.

## Claims

1. An angiographic catheter (10) comprising a composite tube formed by an inner sleeve (20) surrounded by an outer sleeve (18) co-tapered therewith over a part of the length of the catheter (10) such that the overall wall thickness of the composite tube is substantially constant throughout its axial length, characterised in that the inner sleeve (20) is composed of a reinforcing polyamide material jacketed by the outer sleeve (18) of soft elastomeric material, in that the outer sleeve (18) extends beyond a tapered end of the reinforcing polyamide inner sleeve (20) to form an unreinforced distal end portion (12) of soft elastomeric urethane constituting a soft tip, and in that the tapered end of the reinforcing polyamide inner sleeve (20) defines an intermediate zone (14) of decreasing stiffness from a main reinforced length (16) of the catheter (10) to the unreinforced distal end portion (12).

2. An angiographic catheter according to Claim 1, characterised in that the intermediate zone (14) has a length in the region of between 1 and 3 centimetres.

3. An angiographic catheter according to Claim 1 or Claim 2, characterised in that the distal end portion (12) of soft elastomeric urethane constituting the said soft tip has a length of between 1 and 7 millimetres.

4. An angiographic catheter according to any preceding Claim characterised in that there is provided a pattern of holes (34) provided along the length of the catheter or at the tip or distal end portion (12) and intermediate zone (14) of the catheter (10).

## Patentansprüche

1. Angiographiscer Katheter (10) mit einem Rohr, bestehend aus einer inneren Hülse (20), die von einer äußeren Hülse (18) umgeben wird, wobei die innere Hülse (20) und die äußere Hülse (18) über einen Teil der Länge des Katheters (10) konisch zulaufend ausgebildet sind, derart, daß über die axiale Länge die Wandstärke des zusammengesetzten Rohres im wesentlichen konstant ist, dadurch gekennzeichnet, daß die innere Hülse (20) aus einem verstärkten Polyamidwerkstoff besteht und durch die äußere Hülse (18) aus weichem elastomeren Werkstoff umgeben wird, wobei die äußere Hülse (18) sich über ein verjüngend zulaufendes Ende der aus einem verstärkten Polyamid bestehenden inneren Hülse (20) erstreckt, um derart ein unverstärktes distales Endteil (12) aus weichem elastomeren Urethan zu bilden, das ein sanftes Endstück schafft, wobei weiterhin das verjüngende Ende der aus verstärktem Polyamid bestehenden inneren Hülse (20) eine Zwischenzone (14) sich verringernder Steifigkeit schafft ausgehend von einer verstärkten Hauptlänge (16) des Katheters (10) zu dem unverstärkten distalen Endteil (12).

2. Angiographischer Katheter nach Anspruch 1, dadurch gekennzeichnet, daß die Zwischenzone (14) eine Länge zwischen 1 und 3 cm aufweist.

3. Angiographischer Katheter gemäß Anspruch 1 oder 2, dadurch gekennzeichnet, daß das distale Endteil (12) aus weichem elastomeren Urethan das sanfte Endstück bildet und eine Länge von etwa 1 bis 7 mm aufweist.

4. Angiographischer Katheter nach einem oder mehreren der vorhergehenden Ansprüche, gekennzeichnet durch ein Muster von Öffnungen (34) über die Länge des Katheters oder an dem Endstück oder dem distalen Endteil (12) und der Zwischenzone (14) des Katheters (10).

**Revendications**

1. Un cathéter angiographique (10) comprenant un tube composite formé par un manchon interne (20) entouré par un manchon externe (18) co-effilés en cône avec celui-ci sur une partie de la longueur du cathéter (10) de façon telle que l'épaisseur de paroi totale du tube composite soit sensiblement constante suivant toute sa longueur axiale, caractérisé en ce que le manchon interne (20) est composé d'un matériau de polyamide de renfort enveloppé par le manchon externe (18) en un matériau élastomère mou, en ce que le manchon externe (18) s'étend au delà d'une extrémité conique du manchon interne (20) en polyamide de renfort pour former une portion d'extrémité distale (12) non renforcée d'uréthane élastomère mou constituant un bout mou, et en ce que l'extrémité conique du manchon interne (20) en polyamide de renfort définit une zone intermédiaire (14) de rigidité décroissante depuis une longueur principale renforcée (16) du cathéter (10) jusqu'à la portion d'extrémité distale (12) non renforcée.

2. Un cathéter angiographique selon la revendication 1, caractérisé en ce que la zone intermédiaire (14) a une longueur de l'ordre de 1 à 3 centimètres.

3. Un cathéter angiographique selon la revendication 1 ou la revendication 2, caractérisé en ce que la portion d'extrémité distale (12) en uréthane élastomère mou constituant ledit bout a une longueur d'environ 1 à 7 millimètres.

4. Un cathéter angiographique selon l'une quelconque des revendications précédentes, caractérisé en ce qu'il est prévu une répartition de trous (34) suivant la longueur du cathéter ou sur le bout ou à la partie d'extrémité distale (12) et dans la zone intermédiaire (14) du cathéter.

Fig.1

Fig.2

EP 0 144 316 B1